# EUROPÄISCHE PATENTSCHRIFT

(11) **EP 1 872 739 B1**
(45) Veröffentlichungstag und Bekanntmachung des Hinweises auf die Patenterteilung: **02.12.2009**
(21) Anmeldenummer: 06116153.5
(22) Anmeldetag: 27.06.2006
(51) Int. Cl.: A61C 8/00

(54) **Bohrer für die Dentalimplantologie**
Dental implantology drill
Foret utilisé dans l'implantologie dentaire

(43) Veröffentlichungstag der Anmeldung: 02.01.2008
(60) Teilanmeldung: 09170067.4
(73) Patentinhaber: Straumann Holding AG, 4002 Basel (CH)
(72) Erfinder: Kmiecz, André, 2610 Mont-Soleil (CH); Günter, Daniel, 4437 Waldenburg (CH); Memmolo, Marcello, 4450 Sissach (CH)
(74) Vertreter: Modiano, Micaela Nadia

(56) Entgegenhaltungen:
- EP-A2- 0 765 640
- DE-U- 1 883 930
- DE-U1- 20 013 654
- DE-U1-202004 000 723
- GB-A- 2 405 365

## Beschreibung

Die vorliegende Erfindung betrifft allgemein einen verbesserten Bohrer für die Dentalimplantologie und insbesondere einen verbesserten Bohrer für die Dentalimplantologie, bei dem das Aufbohren einer bereits bestehenden Bohrung vereinfacht werden soll.

### Stand der Technik

In der Dentalimplantologie ist es bekannt, für die Fertigstellung von Bohrungen als Implantatbett, einen Satz von Bohrern zu verwenden, umfassend Rosenbohrer, Pilotbohrer und Spiralbohrer. Ein derartiger Satz von Bohrern wird beispielsweise von der Firma Institut Straumann AG, Basel, Schweiz vertrieben.

In groben Zügen ist das Vorgehen bei der Fertigstellung einer Bohrung als Implantatbett wie folgt. Als erstes wird die freigelegte Knochenoberfläche mit einem Rosenbohrer geglättet und die Implantationsstelle wird mit dem Rosenbohrer markiert und möglicherweise erweitert, wobei im letzteren Fall Rosenbohrer mit aufsteigenden Durchmessern eingesetzt werden. Anschließend wird das Implantatbett mit einem geeigneten Pilotbohrer zur Bestimmung der Bohrachse präpariert. In einem abschließenden Schritt wird dann das Implantatbett auf die endgültige erforderliche Breite mit einem geeigneten Spiralbohrer aufgebohrt.

Bei der obigen Vorgehensweise entsteht jedoch beim Aufbohren mit den Spiralbohrer ein Ratteln bzw. Ausschlagen, das durch die Schneidkanten des Spiralbohrers verursacht wird, die in die Kante der vorherigen kleineren Bohrung greifen bzw. in dieser hängen bleiben. Dadurch entsteht das oben erwähnte Ratteln bzw. Ausschlagen des Spiralbohrers.

Dieses Problem wurde bis dato gelöst, indem vor dem Aufbohren mit dem Spiralbohrer die Kanten der kleineren Bohrung mit einem Profilfräser leicht angeschrägt wurden. Die vorstehende Lösung ist jedoch von Nachteil, da sie ein zusätzliches Werkzeug, nämlich einen Profilfräser und einen entsprechenden Arbeitsgang erfordert. Bei einem erneuten Aufbohren auf einem größeren Durchmesser wären entsprechend ein weiterer Profilfräser und Arbeitsgang erforderlich.

Eine Alternative zu der obigen Lösung wird von der WO 2004/080325 A1 angeboten, worin eine Pilotführung mit kleinerem Durchmesser an der Spitze des Spiralbohrers integriert ist. Der kleinere Durchmesser der Pilotführung entspricht dem Durchmesser des vorherigen Pilotbohrers bzw. Pilotbohrung und dient zur besseren Führung des Spiralbohrers.

Die Alternative der WO 2004/080325 A1 ist insofern nachteilig, da sie unnötiges Knochenmaterial an der Pilotführung abträgt, wobei im Implantatbett ein überflüssiges Sackloch apikal gebildet wird, das für die Aufnahme des Dentalimplantats nicht erforderlich ist und der Osteointegration bzw. Einheilung des Implantats abträglich ist. Dieser Nachteil kann zwar durch eine entsprechende Gestaltung der Länge der Bohrung und die Bereitstellung eines zusätzlichen Bohrers zum Abtragen des Knochengewebes im Bereich der Pilotführung gelöst werden, das wiederum zum Problem des zusätzlichen Werkzeugs und eines zusätzlichen Arbeitsgangs führt.

Aus der DE 200 13 654 U1 ist ein Bohrer gemäß Oberbegriff des Anspruchs 1 bekannt.

### Zusammenfassung der Erfindung

Daher ist es eine Aufgabe der vorliegenden Erfindung einen Bohrer für die Dentalimplantologie bereitzustellen, der die obigen Nachteile vermeidet und der ein Aufbohren auf dem Implantatbett ohne zusätzliches Werkzeug und frei von Ratteln bzw. Ausschlagen erlaubt, wobei gleichzeitig eine gute Führung gewährleistet wird.

Diese und weitere der nachstehenden Beschreibung zu entnehmenden Aufgaben werden von einem Bohrer für die Dentalimplantologie gemäß den anliegenden Ansprüchen gelöst.

Weitere Merkmale und Vorteile der vorliegenden Erfindung sowie die Wirkungsweise der exemplarischen Ausführungsform der vorliegenden Erfindung werden unten mit Bezug auf die begleitenden Zeichnungen beschrieben. Die begleitenden Zeichnungen veranschaulichen die vorliegende Erfindung und dienen zusammen mit der Beschreibung weiterhin dazu, die Grundsätze der Erfindung zu erklären und es einem Fachmann auf dem betreffenden Gebiet zu ermöglichen, die Erfindung herzustellen und zu verwenden. Dabei zeigen:
Fig. 1 eine perspektivische Ansicht eines Bohrers für die Dentalimplantologie gemäß einer exemplarischen Ausführungsform der vorliegenden Erfindung;
Fig. 2 eine Seitenansicht des Bohrers für die Dentalimplantologie gemäß der exemplarischen Ausführungsform der Fig. 1; und
Fig. 3 eine Ansicht der Bohrerspitze des Bohrers für die Dentalimplantologie gemäß der exemplarischen Ausführungsform der Fig. 1.

### Beschreibung der bevorzugten Ausführungsformen der Erfindung

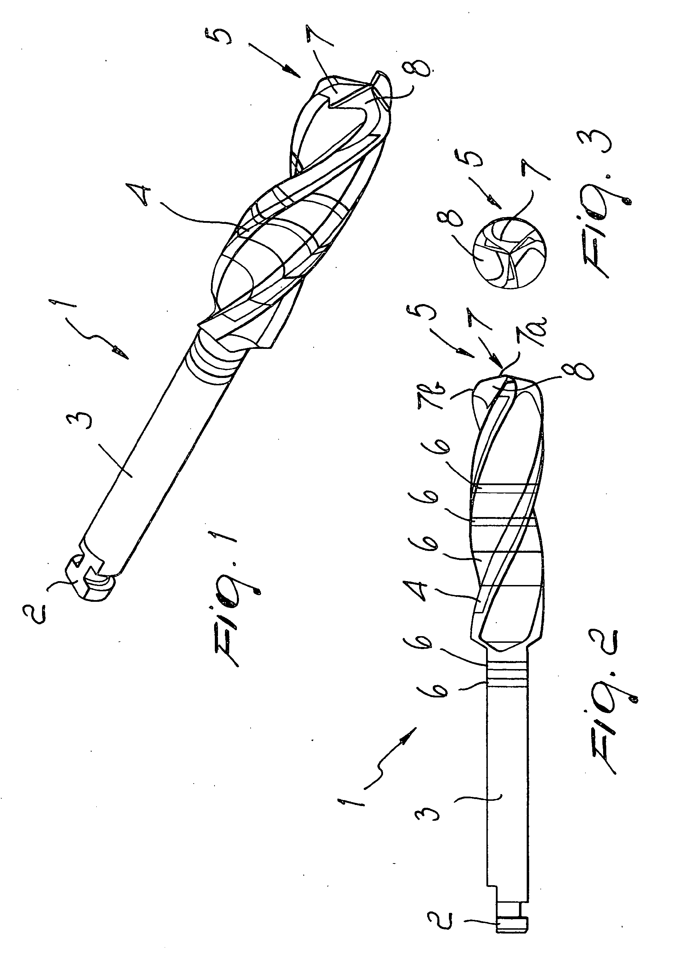

Unter Bezugnahme auf die Figuren 1 bis 3 wird eine derzeit bevorzugte Ausführungsform des erfindungsgemäßen Bohrers 1 gezeigt. Dieser Bohrer kann auf einem Spiralbohrer der Firma Institut Straumann AG, Basel, Schweiz basieren, der in einer 3,5 mm Durchmesser Version kurz oder lang (Art. Nr. 044.218/219) oder in einer 4,2 mm Durchmesser Version kurz oder lang (Art. Nr. 044.222/223) erhältlich ist.

Der Bohrer 1 weist in einer herkömmlichen Art und Weise eine Dentalkupplung 2, einen Bohrerschaft 3, einen Bohrerhals 4 mit mindestens einer Spiralnut und einer entsprechenden Fase, und eine Bohrerspitze 5 mit mindestens einer Spitzenschneide 7 und einem entsprechenden Anschliff 8 auf. Darüber hinaus werden in üblicher Weise mehrere Markierungen 6 auf dem Bohrer 1 zur Erfassung der Bohrtiefe bereitgestellt.

Erfindungsgemäß ist die Spitzenschneide 7 der Bohrerspitze 5 mit Abrundungen 7b und Kanten 7a wie in der Seitenansicht der Fig. 2 zu ersehen, ausgebildet. Die Abrundungen 7b schließen zum Bohrerhals 4 an, so dass der Berührungspunkt der Abrundungen 7b mit dem Bohrerhals 4 dem Durchmesser des Bohrers 1 entspricht. Somit sind die Kanten 7a Teile eines imaginären Kegels, dessen Achse durch die Achse des Bohrers verläuft und die Abrundungen 7b Teile einer imaginären Kugel, deren Mittelpunkt ebenfalls auf der Achse des Bohrers liegt. Vorzugsweise ist der imaginäre Kegel stumpf.

Die Kanten 7a bilden vorzugsweise untereinander einen Winkel von etwa 140° bis etwa 180°, bevorzugter sind etwa 150° bis etwa 174° und am meisten bevorzugt sind etwa 160°.

Vorzugsweise hat die imaginäre Kugel, die durch die Abrundungen 7b gebildet wird und deren Mittelpunkt auf der Achse des Bohrers liegt, einen Radius, der in etwa 1,5 mm bei einem 4,2, mm Spiralbohrer mit der vorstehend angegebenen Bezeichnung beträgt. Der Radius bei einem 3,5 mm Spiralbohrer beträgt ebenfalls etwa 1,5 mm.

Die Erfindung erfüllt gänzlich die gestellten Aufgaben, da ein Bohrer, insbesondere ein Spiralbohrer für die Dentalimplantologie bereitgestellt wird, der trotz fehlender Führungsspitze besonders laufruhig sowie gut zentriebar ist, und das Problem des Rattelns bzw. Ausschlagens vermeidet.

Auch wird die Notwendigkeit eines zusätzlichen Bohrers zur Entfernung des nicht weggebohrten Knochens im Bereich der Pilotführung vermieden, wie im Fall der Lösung gemäß der WO 2004/080325 A1 erforderlich. Darüber hinaus wird der weitere Arbeitsgang zum Einsetzen des zusätzlichen Bohrers vermieden.

Obwohl die Erfindung in Verbindung mit einem Spiralbohrer beschrieben wurde, ist für den Fachmann ohne weiteres verständlich, dass die erfindungsgemäße Bohrerspitze auch an einen Pilotbohrer anlegbar ist, sofern dieser zum Aufbohren einer bereits bestehenden Bohrung eingesetzt wird, wobei selbstverständlich dieselben Vorteile wie im Zusammenhang mit einem Spiralbohrer beschrieben, erzielt werden.

Wenn in irgendeinem der Ansprüche erwähnte technische Merkmale mit einem Bezugszeichen versehen sind, wurden diese Bezugszeichen lediglich eingeschlossen, um die Verständlichkeit der Ansprüche zu erhöhen. Entsprechend haben diese Bezugszeichen keine einschränkende Auswirkung auf den Schutzumfang eines jeden Elements, das exemplarisch durch solche Bezugszeichen bezeichnet wird.

## Patentansprüche

1. Bohrer für die Dentalimplantologie umfassend eine Dentalkupplung (2), einen Bohrerschaft (3), einen Bohrerhals (4), und eine Bohrerspitze 5 mit mindestens einer Spitzenschneide (7), wobei Spitzenschneide (7) zum Teil abgerundet ist, **dadurch gekennzeichnet,**
**dass** die Spitzenschneide (7) Kanten (7a) und zum Bohrerhals (4) anschließenden Abrundungen (7b) umfasst, wobei Kanten (7a) Teile eines imaginären Kegels sind, dessen Achse durch die Achse des Bohrers verläuft, und die Abrundungen (7b) Teile einer imaginären Kugel sind, deren Mittelpunkt ebenfalls auf der Achse des Bohrers liegt.

2. Bohrer für die Dentalimplantologie nach Anspruch 1, **dadurch gekennzeichnet, dass** die Kanten (7a) untereinander einen Winkel von etwa 140° bis etwa 180° bilden.

3. Bohrer für die Dentalimplantologie nach Anspruch 1, **dadurch gekennzeichnet, dass** die Kanten (7a) untereinander einen Winkel von etwa 150° bis etwa 170° bilden.

4. Bohrer für die Dentalimplantologie nach Anspruch 1, **dadurch gekennzeichnet, dass** die Kanten (7a) untereinander einen Winkel von etwa 160° bilden.

5. Bohrer für die Dentalimplantologie nach einem oder mehren der vorangehenden Ansprüche, **dadurch gekennzeichnet, dass** der Bohrer als Spiralbohrer oder Pilotbohrer ausgebildet ist.

## Claims

1. Dental implantology drill comprising a dental coupling (2), a drill shaft (3), a drill neck (4) and a drill bit (5) with at least one bit cutter (7), the bit cutter (7) being partly rounded, **characterized in that**
the bit cutter (7) comprises edges (7a) and roundings (7b) bordering on the drill neck (4), the edges (7a) being parts of an imaginary cone whose axis runs through the axis of the drill, and the roundings (7b) being parts of an imaginary sphere whose center is also located on the axis of the drill.

2. Dental implantology drill according to Claim 1, **characterized in that** the edges (7a) form an angle of approximately 140° to approximately 180° among them.

3. Dental implantology drill according to Claim 1, **characterized in that** the edges (7a) form an angle of approximately 150° to approximately 170° among them.

4. Dental implantology drill according to Claim 1, **characterized in that** the edges (7a) form an angle of approximately 160° among them.

5. Dental implantology drill according to one or more of the preceding claims, **characterized in that** the drill is embodied as a helical drill or a pilot drill.

## Revendications

1. Fraise pour l'implantologie dentaire comportant un couplage dentaire (2), une queue de fraise (3), un col de fraise (4) et un embout de fraise (5) ayant au moins un bord d'embout tranchant (7), le bord d'embout tranchant (7) étant en partie arrondi, **caractérisée en ce que**
le bord d'embout tranchant (7) comporte des bords (7a) et des arrondis (7b) contigus au col de fraise (4), les bords (7a) étant des parties d'un cône fictif dont l'axe passe par l'axe de la fraise, et les arrondis (7b) étant des parties d'un cône fictif dont le centre est également sur l'axe de la fraise.

2. Fraise pour l'implantologie dentaire selon la revendication 1, **caractérisée en ce que** les bords (7a) forment entre eux un angle d'environ 140 ° à environ 180 °.

3. Fraise pour l'implantologie dentaire selon la revendication 1, **caractérisée en ce que** les bords (7a) forment entre eux un angle d'environ 150 ° à environ 170 °.

4. Fraise pour l'implantologie dentaire selon la revendication 1, **caractérisée en ce que** les bords (7a) forment entre eux un angle d'environ 160 °.

5. Fraise pour l'implantologie dentaire selon une ou plusieurs des revendications précédentes, **caractérisée en ce que** la fraise est configurée comme une fraise hélicoïdale ou une fraise pilote.
